(19) Europäisches Patentamt
European Patent Office
Office européen des brevets

(11) **EP 4 674 348 A1**

(12) **EUROPEAN PATENT APPLICATION**

(43) Date of publication:
**07.01.2026 Bulletin 2026/02**

(21) Application number: **25184339.7**

(22) Date of filing: **21.06.2025**

(51) International Patent Classification (IPC):
**A61B 5/346** (2021.01)   **A61B 5/00** (2006.01)

(52) Cooperative Patent Classification (CPC):
**A61B 5/346; A61B 5/7267**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **04.07.2024 IN 202421051386**

(71) Applicant: **Tata Consultancy Services Limited**
**Mumbai, Maharashtra 400 021 (IN)**

(72) Inventors:
• **SHARMA, Varsha**
**452005 Madhya, Pradesh (IN)**
• **GHOSE, Avik**
**700135 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **UNSUPERVISED MODELING OF A DEEP NEURAL NETWORK FOR ANALYZING AGE-RELATED IMPACTS ON ELECTROCARDIOGRAMS**

(57)    This disclosure relates generally to a method and system for analyzing an effect of age-related variations on the electrocardiogram (ECG). State-of-the-art methods have delved into supervised deep-learning approaches based on regression that does not reflect the relation between a chronological age (CA) and a biological age (BA) of the subject. The disclosed method involves an unsupervised learning approach with a three-step training strategy combining model training and deep ECG features clustering with a controlled initialization. A deep learning model is trained to obtain an age-informed convolutional autoencoder network by identifying one or more errors while processing the reconstructed ECG. Combining the CA and ECG, the trained deep learning model reveals the BA of the heart as a contributing biomarker for estimating the overall BA of the body.

FIG. 2

## Description

CROSS-REFERENCE TO RELATED APPLICATIONS AND PRIORITY

The present application claims priority from Indian provisional application no. 202421051386, filed on July 04, 2024.

TECHNICAL FIELD

The disclosure herein generally relates to cardiac signal analysis, and, more particularly, a method and system for a neural network assisted electrocardiogram (ECG) analysis to examine age-related impacts.

BACKGROUND

Understanding and assessing biological age (BA) is paramount in healthcare and personalized medicine. While chronological age (CA) indicates the number of years lived, the BA reflects an individual's overall health and well-being. By considering various physiological markers, health parameters, and lifestyle factors, BA captures the subtle changes occurring within the body, offering insights into the rate of ageing and potential health risks. This information becomes a powerful tool for healthcare practitioners in tailoring interventions, preventative strategies, and treatment plans based on an individual's unique ageing trajectory. BA is also crucial in insurance contexts. By incorporating lifestyle and health metrics, insurers can more accurately predict health risks, enabling personalized and fair premium pricing. By adopting a preventive strategy and refining risk models, insurance companies can encourage policyholders to maintain better health and incentivize healthier living, potentially reducing long-term healthcare costs and contributing to a more sustainable insurance system. An electrocardiogram (ECG) is an insightful signal for predicting BA. As the heart ages, rhythm, conduction, and overall cardiac function changes become evident. Moreover, ECG's noninvasive nature and widespread availability make it an accessible and valuable tool for assessing biological age. The biophysics of age is intricately entwined with ECG. ECG tracks the natural decline in heart rate due to changes in pacemaker cells and our autonomic nervous system. The ECG can also help identify irregular heart rhythms and conditions like atrial fibrillation. The age-related transformations in the heart's conduction system and structural composition manifest in ECG data in terms of Heart Rate Variability, P-Wave Duration, QRS Complex Duration, QT Interval, PR Interval, ST Segment Changes, and T-Wave Morphology, providing a comprehensive snapshot of the biophysics of age, more specifically, heart age.
Numerous studies have delved into supervised deep-learning approaches for ECG analysis, particularly in predicting age. These studies rely on regression-based methods and necessitate additional analysis for extracting health-related insights, such as the correlation of error between the chronological age and the AI-predicted age with mortality. The existing supervised training algorithms considers the CA as a ground truth without any follow-up data. This type of training forces the network to produce output similar to the CA. Instead, the goal is to study the relationship between BA and CA concerning ECG. It is known that for healthy people, BA is less than equal to CA and otherwise for unhealthy people. Hence, it is said that the relation between BA and CA is proportional but not always linear. Further, supervised learning is only suitable for some individuals (healthy). However, others (who have a medical condition) expect a significant deviation in BA from the CA. Therefore, these regression-based approaches result in an MAE (between BA and CA) in the 5 to 10-year range. This value of MAE is consistent in all supervised learning approaches, irrespective of the network. However, this MAE value does not reflect the actual relationship between BA and CA, which is associated with the progression of ECG. Therefore, employing unsupervised learning or an indirect approach to analyze the effect of age on ECG is necessary.

SUMMARY

Embodiments of the present disclosure present technological improvements as solutions to one or more of the above-mentioned technical problems recognized by the inventors in conventional systems. For example, in one embodiment, a method for training a DL model for analyzing the health of a subject based on effect of the age on the ECG of the subject is provided. The method includes receiving a set of pre-processed ECG of a subject by a Deep Learning (DL) model wherein the DL model is an autoencoder comprising an encoder, and a decoder. The pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time. The method further includes training the DL model to generate a trained DL model by optimizing (a) one or more losses of an autoencoder enabled encoding-decoding, (b) one or more errors of age prediction, and (c) one or more errors of cluster prediction, by a three-step training. In the first step of the three-step training, a reconstruction loss associated with obtaining a first reconstructed ECG for the set of pre-processed ECG is optimized by iteratively updating randomly initialized one or more weights of the encoder. To obtain the first reconstructed ECG, the set of pre-processed ECG is encoded to a first set of latent code by the encoder, and the first set of latent code is decoded by the decoder. In the second step of the three-step training, a reconstruction loss and an error of age prediction associated with obtaining a second reconstructed ECG are optimized by iteratively updating the one or more weights of the encoder. The second reconstructed ECG is obtained by (a) re-initializing the one or more weights of the encoder from the first set of latent code to encode an age-informed second set of latent code; and (b) decoding the age-informed second set of latent code by the decoder, and wherein the age information is processed by a sigmoid layer. A reconstruction loss as well as an error in age prediction associated with obtaining the second reconstructed ECG for the set of pre-processed ECG is optimized by iteratively updating, the re-initialized one or more weights of the encoder. The age information is brought into the autoencoder by adding a sigmoid layer next to the first set of latent code thereby training in the direction of age prediction, making the encoder age-informed. In the third step of the three-step training, the reconstruction loss and an error of cluster prediction associated with obtaining (a) a third reconstructed ECG, and (b) one or more cluster centers, are optimized by iteratively updating the one or more weights of the encoder. The third reconstructed ECG and the one or more cluster centers comprises re-initializing the one or more weights of the encoder from the age-informed second set of latent code for encoding the age-informed second set of latent code. The error of cluster prediction is associated with the extraction of the one or more clusters from the set of pre-processed ECG by a k-means clustering layer. The optimized reconstruction loss, the optimized error of age prediction, and the optimized error of cluster prediction causes reduction in total loss in training the DL model. The sigmoid layer added to the encoder in the second step is replaced by the k-means clustering layer that generates the one or more clusters. The optimized reconstruction loss, the optimized error of age prediction, and the optimized error of cluster prediction causes reduction in a total loss in training the DL model.

In another aspect, a system a method for training a DL model for analyzing the health of a subject based on effect of the age on the ECG of the subject is provided. The system includes at least one memory storing programmed instructions; one or more Input /Output (I/O) interfaces; and one or more hardware processors, a domain aware model, operatively coupled to a corresponding at least one memory, wherein the system is configured to receive a set of pre-processed ECG of a subject by a Deep Learning (DL) model wherein the DL model is an autoencoder comprising an encoder, and a decoder. The pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time. The system is configured to train the **DL** model to generate a trained **DL** model by optimizing by optimizing (a) one or more losses of an autoencoder enabled encoding-decoding, (b) one or more errors of age prediction, and (c) one or more errors of cluster prediction, by a three-step training. **In** the first step of the three-step training, a reconstruction loss associated with obtaining a first reconstructed ECG for the set of pre-processed ECG is optimized by iteratively updating randomly initialized one or more weights of the encoder. To obtain the first reconstructed ECG, the set of pre-processed ECG is encoded to a first set of latent code by the encoder, and the first set of latent code is decoded by the decoder. In the second step of the three-step training, a reconstruction loss and an error of age prediction associated with obtaining a second reconstructed ECG are optimized by iteratively updating the one or more weights of the encoder. The second reconstructed ECG is obtained by (a) re-initializing the one or more weights of the encoder from the first set of latent code to encode an age-informed second set of latent code; and (b) decoding the age-informed second set of latent code by the decoder, and wherein the age information is processed by a sigmoid layer. A reconstruction loss as well as an error in age prediction associated with obtaining the second reconstructed ECG for the set of pre-processed ECG is optimized by iteratively updating, the re-initialized one or more weights of the encoder. The age information is brought into the autoencoder by adding a sigmoid layer next to the first set of latent code thereby training in the direction of age prediction, making the encoder age-informed. In the third step of the three-step training, the reconstruction loss and an error of cluster prediction associated with obtaining (a) a third reconstructed ECG, and (b) one or more cluster centers, are optimized by iteratively updating the one or more weights of the encoder. The third reconstructed ECG and the one or more cluster centers comprises re-initializing the one or more weights of the encoder from the age-informed second set of latent code for encoding the age-informed second set of latent code. The error of cluster prediction is associated with the extraction of the one or more clusters from the set of pre-processed ECG by a k-means clustering layer. The optimized reconstruction loss, the optimized error of age prediction, and the optimized error of cluster prediction causes reduction in total loss in training the DL model. The sigmoid layer added to the encoder in the second step is replaced by the k-means clustering layer that generates the one or more clusters. The optimized reconstruction loss, the optimized error of age prediction, and the optimized error of cluster prediction causes reduction in a total loss in training the DL model.

In yet another aspect, a computer program product including a non-transitory computer-readable medium embodied therein a computer program a method for training a DL model for analyzing the health of a subject based on effect of the age on the ECG of the subject is provided. The computer readable program, when executed on a computing device, causes the computing device to receive, via one or more hardware processors, a set of pre-processed ECG of a subject by a Deep Learning (DL) model wherein the DL model is an autoencoder comprising an encoder, and a decoder.

The pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time. The computer readable program, when executed on a computing device, causes the computing device to train the DL to generate a trained DL model by optimizing (a) one or more losses of an autoencoder enabled encoding-decoding, (b) one or more errors of age prediction, and (c) one or more errors of cluster prediction, by a three-step training. In the first step of the three-step training, a reconstruction loss associated with obtaining a first reconstructed ECG for the set of pre-processed ECG is optimized by iteratively updating randomly initialized one or more weights of the encoder. To obtain the first reconstructed ECG, the set of pre-processed ECG is encoded to a first set of latent code by the encoder, and the first set of latent code is decoded by the decoder. In the second step of the three-step training, a reconstruction loss and an error of age prediction associated with obtaining a second reconstructed ECG are optimized by iteratively updating the one or more weights of the encoder. The second reconstructed ECG is obtained by (a) re-initializing the one or more weights of the encoder from the first set of latent code to encode an age-informed second set of latent code; and (b) decoding the age-informed second set of latent code by the decoder, and wherein the age information is processed by a sigmoid layer. A reconstruction loss as well as an error in age prediction associated with obtaining the second reconstructed ECG for the set of pre-processed ECG is optimized by iteratively updating, the re-initialized one or more weights of the encoder. The age information is brought into the autoencoder by adding a sigmoid layer next to the first set of latent code thereby training in the direction of age prediction, making the encoder age-informed. In the third step of the three-step training, the reconstruction loss and an error of cluster prediction associated with obtaining (a) a third reconstructed ECG, and (b) one or more cluster centers, are optimized by iteratively updating the one or more weights of the encoder. The third reconstructed ECG and the one or more cluster centers comprises re-initializing the one or more weights of the encoder from the age-informed second set of latent code for encoding the age-informed second set of latent code. The error of cluster prediction is associated with the extraction of the one or more clusters from the set of pre-processed ECG by a k-means clustering layer. The optimized reconstruction loss, the optimized error of age prediction, and the optimized error of cluster prediction causes reduction in total loss in training the DL model. The sigmoid layer added to the encoder in the second step is replaced by the k-means clustering layer that generates the one or more clusters. The optimized reconstruction loss, the optimized error of age prediction, and the optimized error of cluster prediction causes reduction in a total loss in training the DL model.

It is to be understood that both the foregoing general description and the following detailed description are exemplary and explanatory only and are not restrictive of the invention, as claimed.

BRIEF DESCRIPTION OF DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system 100 for analysing an effect of age on electrocardiogram (ECG), according to some embodiments of the present disclosure.
FIG. 2 is an illustrative flow diagram involving components for training a Deep Learning (DL) model for analyzing the ECG, according to some embodiments of the present disclosure.
FIG. 3 is a flow diagram of an illustrative method 300 for training a DL model for analysing an effect of age on the ECG, according to some embodiments of the present disclosure.
FIG. 4 is an architecture of the ECG signal based autoencoder, according to some of the embodiments of the present invention.
FIGS. 5A and 5B are a graphical visualization of an age-informed latent code of cluster centroids and the reconstructed signal from the age-informed latent code respectively, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. Majority of the work has been done on age prediction using a 12 lead ECG. E.g., in one prior art reference, a convolutional neural network (CNN) model has been trained on 10 seconds of 12-lead ECG to predict age and sex and received an average age error of $6.9 \pm 5.6$ years. In this study, a substantially large dataset of adult patients is utilized with follow-up signals after 23 years collected by the Mayo Clinic. Similarly, in other work, the dataset with a follow-up of $12.4 \pm 5.3$ years is utilized in training the same network to find an age gap of 0.88±7.4 years. Further, a correlation between the age gap with total and cardiovascular mortality is explored. Few more studies wherein a unidimensional residual network is trained on the 12-lead ECG from multiple datasets and concluded that patients with AI-predicted ECG age more than eight years greater than their chronological age (CA). Further, Inception time, Bi-LSTM, CNN, and ResNeXt (a modified ResNet) architectures, have been explored to gain an insight of the health by predicting biological age utilizing ECG. Most BA prediction techniques in the literature utilizes deep learning networks for supervised training, in which the CA is considered the ground truth without any follow-up data. This type of training forces the network to produce output similar to the CA. Instead, the goal is to study the relationship between the BA and the CA concerning the ECG. It is known that for healthy people, the BA is either less or equal to the CA and other way round in case of unhealthy people. Therefore, it can be asserted that the relation between the BA and the CA is proportional but not always linear. The supervised learning method are seen to result in a mean absolute error (MAE) between BA and CA in the range of 5-10 years. This value of MAE is consistent in all the supervised learning approaches, irrespective of the network. Therefore, in the present invention, an unsupervised learning is adopted to analyze the effect of chronological age on the ECG. The disclosed deep learning (DL) model receives the CA and the ECG of the subject to predict health of the subject which is an indicator of the biological age of the subject.

As used herein the term "biological age" refers to an authentic ageing progression of an individual in relation to their quality of life.

As used herein the term "chronological age" the number of years lived.

As used herein the term "random initialization" refers to selection of initial values randomly.

As used herein the term "re-initialization" refers to selection of initial values from the age-informed latent space.

As used herein the terms "subject", "human", "individual" are used interchangeably throughout the specification and refers to a human being undergoing health analysis based on his/her ECG given as input to a deep learning model.

As used herein the terms "Deep Learning (DL) model" and "DL model" are used interchangeably throughout the specification and refers to a multilayered neural network model.

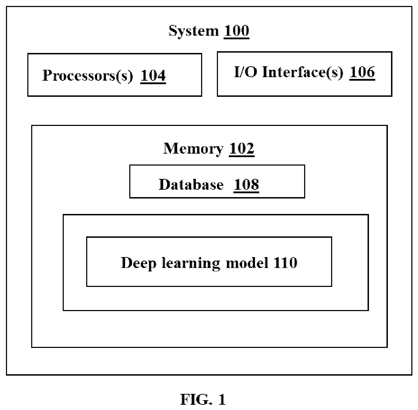

Referring now to the drawings, and more particularly to FIG. 1 through FIG. 5B, where similar reference characters denote corresponding features consistently throughout the figures, there are shown preferred embodiments, and these embodiments are described in the context of the following exemplary system and/or method.

FIG. 1 illustrates an exemplary block diagram of a system 100 for analysing an effect of age on electrocardiogram (ECG), according to some embodiments of the present disclosure.

In an embodiment, the system 100 includes a processor(s) 104, communication interface device(s) 106, alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100. Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like. The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to number of external devices or to another server or devices. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 includes a deep learning (DL) model 110. The DL model 110 employs a two-dimensional (2D) convolutional autoencoder (herein named BioAgeNet™) to learn a feature space associated with age. The 2D convolutional autoencoder comprises an encoder and a decoder. The encoder maps the input data to representation space to extract the latent features and the decoder reconstructs the data from representation space to the input space. The encoder part comprises five 2D convolutional layers (Conv2D), and the decoder comprises a plurality of transposed Conv2D (Conv2D Trans) layers. The 2D convolutional autoencoder returns one or more cluster centroids that indicate two states: (i) healthy, and (ii) unhealthy. Therefore, the subject undergoing evaluation of health by the disclosed 2D convolutional autoencoder, is classified as either health or unhealthy. This classification is generated based on age-informed features that represents quality of lifestyle an individual follows. The memory 102 further comprises of a plurality of modules that includes programs or coded instructions that supplement applications or functions performed by the system 100 for executing different steps involved in the analysis of the health of the subject, being performed by the system 100. The modules, amongst other things, can include routines, programs, objects, components, and data structures, which perform particular tasks or implement particular abstract data types. The modules may also be used as signal processor(s), node machine(s), logic circuitries, and/or any other device or component that manipulates signals based on operational instructions. Further, the modules can be used by hardware, by computer-readable instructions executed by the one or more hardware processors 104, or by a combination thereof. The modules may include computer-readable instructions that supplement applications or functions performed by the system 100. Further, the memory 102 may comprise information pertaining to input(s)/output(s) of each step performed by the processor(s) 104 of the system100 and methods of the present disclosure. Further, the memory 102 includes a database 108. The database (or repository) 108 may include a plurality of abstracted piece of code for refinement and data that is processed, received, or generated as a result of the execution of the plurality of modules. The external database is communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

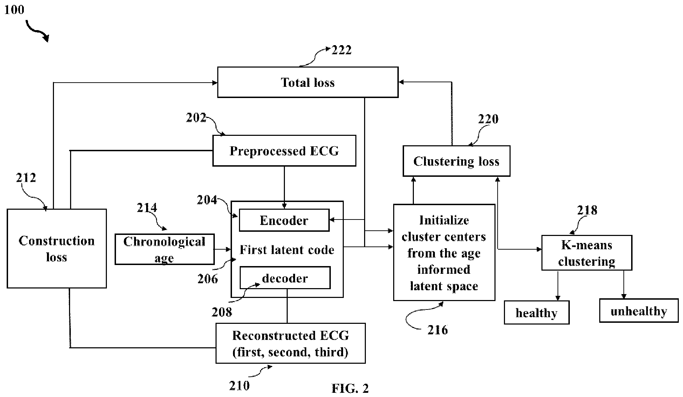

FIG. 2 is an illustrative flow diagram involving components for training a Deep Learning (DL) model for analyzing the ECG, according to some embodiments of the present disclosure.

As illustrated in the FIG. 2, the DL model capable of processing the ECG signal to analyse health of the subject is presented. The DL model is an autoencoder neural network (alternately referred to as 'autoencoder'). The autoencoder is an unsupervised machine learning algorithm that applies backpropagation, setting the target values to be equal to the inputs. The DL model training involves a three-step process that utilizes a plurality of autoencoder components and also balances an encoding-decoding loss occur during each step of the training. The autoencoder comprises an encoder 204 and a decoder 208. In the first step, the pre-processed ECG signal 202 is given to the autoencoder wherein the encoder 204 encodes the pre-processed ECG as a first set of latent code 206. The first set of latent code 206 is a compressed representation of the pre-processed ECG in a reduced dimension. The decoder 208 receives the first set of latent code 206 and decode it to the reconstructed ECG signal 210. The encoding and decoding processes results in a construction loss 212. The construction loss is optimized by continuously updating one or more weights of the encoder 204. In the second step, the chronological age 214 is provided to the first set of latent code. The autoencoder again receives the pre-processed ECG signal 202 and the one or more weights of the encoder 204 and re-initializes the one or more weights of the encoder 204 from the first set of latent code to encode the pre-processed ECG signal 202 into an age-informed second set of latent code, and wherein the age-informed second set of latent code are decoded to obtain a second reconstructed ECG signal. Further one or more cluster centers 216 are initialized from the age-informed second set of latent code by encoding the pre-processed ECG signal 202 to obtain an age-informed third set of latent code, and wherein the age-informed third set of latent code are decoded to obtain a third reconstructed ECG signal. In the third step, a k-means clustering 218 is performed by introducing the k-means clustering layer to the autoencoder. The k-means clustering layer segregates a plurality of cluster centroids from the age-informed second set of latent code, and further groups one or more similar age-informed third latent code by forming the plurality of cluster centroids and wherein the plurality of cluster centroids represents a health state of the subject. K-means clustering utilizes a pre-defined criteria specified at the start of the model training. Based on the pre-defined clustering criteria, the heath state of the subject is classified as "healthy" and "unhealthy". The training considers the clustering loss 220 and optimizes the clustering loss by continuously updating the one or more weights of the encoder processing the pre-processed ECG signal in the third step. The training enables simultaneous minimization of a mean absolute error of reconstruction loss ($MAE_{Recon}$) and a mean absolute cluster centroids error ($MAE_{Clust}$) to encourage the k-means clustering algorithm. The total loss 222 aggregates the reconstruction loss of updating weights and clustering loss of updating cluster centers.

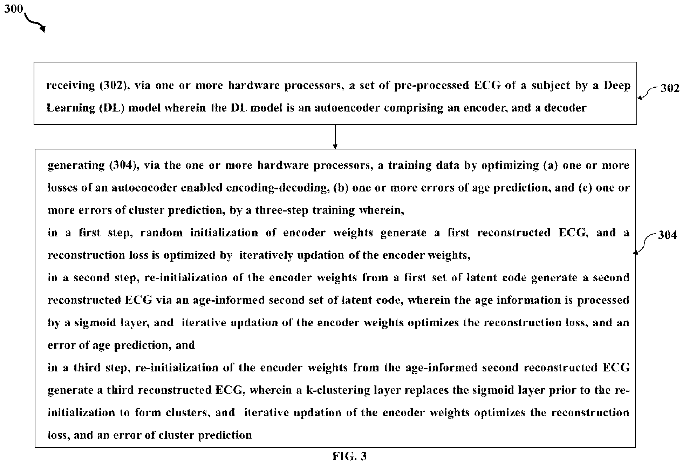

FIG. 3 is a flow diagram of an illustrative method 300 for training a DL model for analysing an effect of age on the ECG, according to some embodiments of the present disclosure.

The steps of the method 300 of the present disclosure will now be explained with reference to the components or blocks of the system 100 as depicted in FIG. 1 through FIG. 5B. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously. In the present disclosure, a systematic approach of training the DL model 110 is employed wherein the entire model training is executed in three steps. And each step is dependent on the previous step in terms of input and output such that the processed output of the first step is utilized as an input in the second step.

At step 302 of the method 300, the one or more hardware processors 104 are configured to receive a set of pre-processed ECG (alternately referred as 'ECG signals') of a subject by a Deep Learning (DL) model wherein the DL model is an autoencoder comprising an encoder, and a decoder. The DL model 110 receives the set of pre-processed ECG. The pre-processing involves data cleansing and data normalization. A channel-wise normalization of the ECG is performed using min-max normalization through one or more hyperparameters. The min-max normalization is a technique that rescales the data values to a range between 0 and 1, using the minimum and maximum values of the original data. This technique preserves the relative order and distance of the data points, but it also reduces the variance and magnifies the effect of outliers. The ECG are further segmented into smaller window of time with a suitable overlap to make the dataset deep-learning network-ready.

At step 304 of the method 300, the one or more hardware processors 104 are configured to train the DL model to generate a trained DL model by optimizing (a) one or more losses of an autoencoder enabled encoding-decoding, (b) one or more errors of age prediction, and (c) one or more errors of cluster prediction, by a three-step training. In a first step of the three-step training process, a reconstruction loss associated with obtaining a first reconstructed ECG for the set of pre-processed ECG is optimized by iteratively updating randomly initialized one or more weights of the encoder. To obtain the first reconstructed ECG, the set of pre-processed ECG is encoded to a first set of latent code by the encoder, and the first set of latent code is decoded by the decoder. The one or more weights of the encoder are randomly initialized to obtain a first set of latent code. The first set of latent code is then decoded by a decoder to generate a first reconstructed ECG. The reconstruction loss associated with obtaining the first reconstructed ECG for the set of pre-processed ECG is optimized by iteratively updating randomly initialized one or more weights of the encoder. The random initialization of the encoder weights ensure a good, reconstructed signal that indicates the required latent features for the next step. In a second step of the three-step training, a reconstruction loss and an error of age prediction associated with obtaining a second reconstructed ECG are optimized. A second reconstructed ECG is obtained by re-initializing the one or more weights of the encoder from the first set of latent code to encode an age-informed second set of latent code. The age-informed second set of latent code is then decoder by the decoder to the second reconstructed ECG. The re-construction loss of generating the second reconstructed ECG is optimized by iteratively updating the one or more weights of the encoder. Prior to the re-initialization, a sigmoid layer in the encoder of the DL model is added. In this step, the tuned DL model is trained by adding a sigmoid layer after the latent code layer to predict age. The encoder weights are initialized at the first epoch from the latent space generated after the first step of training and then the encoder weights are continuously updated with the optimization of the combined loss ($CL_1$). The $CL_1$ is represented in equation (1). During the second step, the model trains the input ECG and generates two outputs: (i) encoded ECG (12000), and (ii) age predictions. The sole aim of training the DL model 110 is to simultaneously minimize mean absolute age error ($MAE_{Age}$) and mean absolute reconstruction error ($MAE_{Recon}$). This creates a feature space represented by the second set of latent code that is age-informed. This means that the set of latent code constitutes the age dependent features. Therefore, in the second step, re-initializing the one or more weights of the encoder from the first set of latent code outputs the age-informed second set of latent code representing a second reconstructed ECG. Here, the one or more weights of the encoder of the DL model 110 are iteratively updated by optimizing combination of a age prediction error and the reconstruction loss. In a third step of the three-step training, the reconstruction loss and an error of cluster prediction associated with obtaining (a) a third reconstructed ECG, and (b) one or more cluster centers, are optimized. The third re-constructed ECG is obtained by re-initializing the one or more weights of the encoder from the age-informed second set of latent code for encoding the age-informed second set of latent code. The error of cluster prediction is associated with the extraction of the one or more clusters from the set of pre-processed ECG by a k-means clustering layer. This results in one or more cluster centroids representing the third reconstructed ECG. The one or more weights of the encoder of the DL model and one or more weights of the cluster centroids are iteratively updated by optimizing a cluster centroid error and the reconstruction loss. To perform clustering, the sigmoid layer added in the previous step in the encoder of the DL model is replaced by a K-means clustering layer. The latent code generated by the encoder are used by a K-means layer to initialize one or more the cluster centroids in a first epoch. A plurality of epochs are carried out, and a cluster assignment is performed in the final epoch. In the cluster assignment, the one or more cluster centroids are clubbed together based on distance between the two centroids to form one or more clusters. The decoder is a transposed Conv2D (Conv2D Trans) comprises a plurality of layers. The Conv2D architecture transposes the input pre-processed ECG, and output reconstructed ECG. The encoder weights are initialized at the first epoch from the age-informed latent space generated after the second step training and then continuously updated with the optimization of the combined loss ($CL_2$) represented in the equation (1). The initialization of the cluster centroids and subsequent clustering in the K-means layer plays a significant role in producing optimal clusters. The training enables simultaneous minimization of a mean absolute error of reconstruction loss ($MAE_{Recon}$) and a mean absolute cluster centroids error ($MAE_{Clust}$) to encourage the k-means clustering algorithm. The optimized reconstruction loss, the optimized error of age prediction, and the optimized error of cluster prediction causes reduction in a total loss in training the DL model. The total loss is an aggregation of the chronological age error, the cluster centroid error and the reconstruction loss. The $CL_1$ and $CL_2$ of the equation (1) together constitute the total loss. Therefore, the total loss is a combination of a first combined loss and a second combined loss, and wherein the first combined loss is a summation of the reconstruction loss of the second reconstructed ECG and the error of age prediction, and the second combined loss is a summation of the reconstruction loss of the third reconstructed ECG and the error of cluster prediction, and wherein the total loss is represented by an equation (1) as:

$$CL_1 = (1 - \lambda_1)MAE_{Recon} + \lambda_1 MAE_{Age}$$
$$CL_2 = (1 - \lambda_2)MAE_{Recon} + \lambda_2 MAE_{Clust} \qquad\qquad (1)$$

wherein,

$(1 - \lambda_1)MAE_{Recon}$ is a mean absolute error of reconstruction of the second reconstructed ECG,
$\lambda_1 MAE_{Age}$ is a mean prediction error of age,
$(1 - \lambda_2)MAE_{Recon}$ is a mean absolute error of reconstruction of the third reconstructed ECG, and
$\lambda_2 MAE_{Clust}$ a mean absolute cluster centroids error ($MAE_{Clust}$).

The third step returns the optimized cluster centroids. The k-means cluster algorithm group similar age-informed latent code vectors and update the cluster centroids during training to refine the groupings. The combined loss calculation involved grid search wherein $\lambda_1$ and $\lambda_2$ are tweaked incrementally from 0.1 to 0.9 with 0.1 unit increment each epoch. The trained DL model is selected that achieved the lowest loss at the epoch for each set of parameters. The trained DL model returns one or more cluster assignments indicating health of the subject. The reconstruction loss of each step and one or more errors at each step are aggregated together to calculate a combined loss. The combined loss is then subjected to optimization. An adam optimizer is utilized to optimize the combined loss of the three-step training process. The adam optimizer utilizes a set of default parameters. Learning rate in the adam optimizer is configurable. However, various other available optimizers can be used to optimize the combined loss. In an embodiment, the learning rate is set to $10^{-4}$. The learning rate is reduced significantly when the validation loss fails to improve over a plurality of consecutive epochs. In an embodiment, the learning rate is reduced by 0.1 when the validation loss fails to improve over three consecutive epochs. The three-step training resulted in a trained DL model that is capable of analyzing health state of the subject on the basis of input ECG of the subject as well as by considering the chronological age of the subject. In the first step of the training, only the reconstruction loss ($MAE_{Recon}$) have been minimized and thus obtained the first reconstructed signal. In the second step, $CL_1$ of the equation (1) have been minimized and second reconstructed signal is obtained with supervised predictions for age. In the third step, the cluster centroids have been initialized from the second latent space generated in the second step. Finally, the $CL_2$ of the equation (1) have been minimized to obtain the third reconstructed signal with the optimized cluster centroids based on input chronological age. The disclosed invention performs three-step training of the DL model 110 . At each step of the training, a reconstruction loss is minimized followed by error minimization. This involves several iterations at each step that involves weight initialization of the one or more weights of the encoder.

The reconstruction loss of each step and one or more errors at each step are aggregated together to calculate a combined loss. The combined loss is then subjected to optimization. An adam optimizer is utilized to optimize the combined loss of the three-step training process. The adam optimizer utilizes a set of default parameters. Learning rate in the adam optimizer is configurable. In an embodiment, the learning rate is set to $10^{-4}$ . The learning rate is reduced significantly when the validation loss fails to improve over a plurality of consecutive epochs. In an embodiment, the learning rate is reduced by 0.1 when the validation loss fails to improve over three consecutive epochs. In the present disclosure, a systematic approach of training the DL model 110 is employed wherein the entire model training is executed in three steps. And each step is dependent on the previous step in terms of input and output such that the processed output of the first step is utilized as an input in the second step.

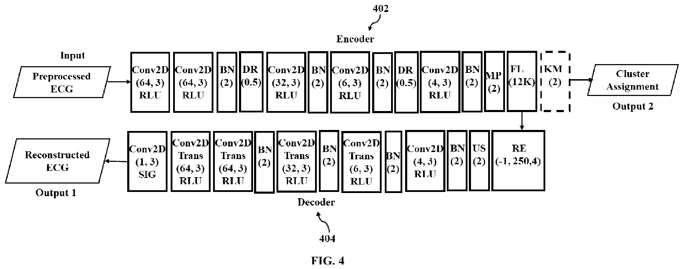

FIG. 4 is an architecture of the ECG signal based autoencoder, according to some of the embodiments of the present invention.

As illustrated in the FIG. 4, the two-dimensional (2D) convolutional autoencoder learns a plurality of extracted features associated with the chronological age of the subject. As these groups are generated based on age-informed features, they represent the quality of lifestyle people follow throughout their chronological age. The convolutional autoencoder comprises an encoder 204 and a decoder 208. The preprocessed ECG signal of shape $12\times500\times1$ is the input to the encoder 402 comprising five 2D-convolutional layers (Conv2D). The initial two Conv2D layers are followed by a batch normalization (BN) and a dropout (DR), a third Conv2D is followed by only BN, a fourth Conv2D is again followed by the BN and the DR, and a fifth Conv2D is Max-pooled (MP) after BN. The MP layer is then flattened to generate a 1D latent code. The latent code is used by the K-means layer to initialize the cluster centroids in the first epoch and then returns the cluster assignments in the final epoch. The second part, the decoder 404, comprises a transposed Conv2D (Conv2D Trans), as this part aims to reconstruct the input ECG. The first layer in the decoder 404 is a reshape (RE), then an Upsample2D (US). Later layers are opposite to the encoder 402, except the dropout layer, which is not present in the decoder 402. The final layer is Conv2D with only one filter of size three and a sigmoid (SIG) activation function to generate reconstruction. The intermediate nonlinearity is implemented using a rectified linear activation unit (RLU).

PERFORMANCE EVALUATION OF CLUSTER CENTRIODS

A comparative evaluation is performed to understand the effectiveness of the three-step training process wherein the ECG as well as the chronological age are given as input to the DL model. In the first scenario, training of DL model is performed by providing the pre-processed ECG to the autoencoder and a reconstructed ECG are obtained after 30 epochs. Whereas in another scenario, the DL model training is performed as per the disclosed three-step process. For both scenarios, cluster quality is evaluated using Calinski-Harabasz Index (CHI) and Davies-Bouldin Index (DBI) scores and results are presented in Table 1. In the first scenario, clusters are not initialized with age-informed latent code, and in the second scenario, clusters are initialized with the age-informed latent code. A need for high-quality clusters and accurate reconstruction in selecting the optimal parameter set of $\lambda_1$ and $\lambda_2$ is managed in both the scenarios. A high performance of CHI and DBI with the value of $\lambda_1$ and $\lambda_2$ as 0.2 and 0.4, respectively are obtained. Higher CHI and Lower DBI represents the higher quality of clusters. Therefore, it has been observed that the CHI is higher and the DBI is lower for age-initialized clusters. Therefore, the second scenario is considered as most suited while analyzing health state of the subject on the basis of ECG.

Table-1

| Cluster quality indexes | Scenario 1 (Reconstruction loss+ without age initialized cluster optimization) | Scenario 2 (Reconstruction loss+ age initialized cluster optimization) |
|---|---|---|
| CHI | 56.32 | 138.47 |
| DBI | 4.34 | 1.29 |

USE CASE:

An example scenario analysing the health state of the subject by the disclosed method utilizing the system 100 is presented below. Also, a comparative analysis of accuracy between the predictions made by the age-informed autoencoder using the disclosed system 100 with other conventional autoencoder is presented below.

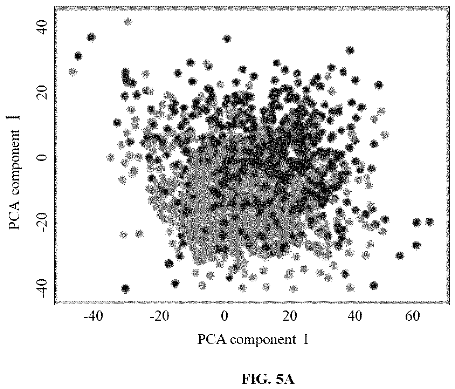

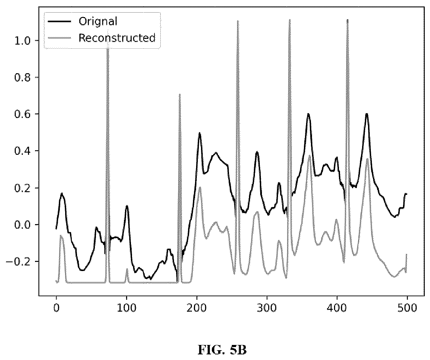

To train a DL model that is able to accurately analyze health state of the subject, a PTB-XL dataset of ECG comprising 12-lead ECG recordings of 10 seconds duration from 21837 subjects at 100 and 500Hz is taken. The dataset is then taken for pre-processing to obtain pre-processed ECG. The pre-processing involves data cleansing and data normalization. The data cleansing involves removing the ECG where age and super diagnosis of the subjects is not present. The ECG are selected at 100 Hz. The channel-wise normalization of the ECG is performed using min-max normalization. The ECG, each having 10 seconds are segmented into 5 second windows with 2.5 seconds of overlap to make the dataset deep-learning network-ready. The pre-processed ECG of shape $12 \times 500 \times 1$ is the input to the five layered 2D convolutional encoder (Conv2D). The training is conducted in three steps. In the first step, the encoder weights are randomly initialized to encode the pre-processed ECG to obtain a first set of latent code. And the encoder weights are iteratively updated by optimizing a reconstruction loss. In the second step, the encoder weights are re-initialized from the first set of latent code to obtain an age-informed second set of latent code representing a second reconstructed ECG and encoder weights are iteratively updated by optimizing the combination of a chronological age error and the reconstruction loss. This represents a DL model predicted age. In the third step, the encoder weights are re-initialized from the second set of latent code to obtain cluster centroids representing a third reconstructed ECG, and the cluster centroids are iteratively updated by optimizing a cluster centroid error and the reconstruction loss using a K-means clustering layer. The mean absolute error (MAE) between the model predicted age at the step two and the input chronological age (CA) is $8.56 \pm 6.89$, and R2 is 0.72. Principal Component Analysis (PCA) is further applied to the latent code represented as clusters if shown in FIG. 5A and the reconstructed signal from the age-informed latent code are shown in FIG. 5B.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the present disclosure or if they include equivalent elements with insubstantial differences from the literal language of the embodiments described herein.

Therefore, the three-step training method utilizing an unsupervised learning technique to explore the potential of electrocardiogram (ECG) data is presented. The three-step training methodology involves the model training, and the unsupervised clustering concurrently. The age-Informed convolutional autoencoder network demonstrates its potential in clustering 12-lead ECG, showcasing its effectiveness in assessing the biological age of the subject. The present disclosure provides an effective means of overcoming the challenge of supervised learning and regression-based methods in age prediction. The convolutional autoencoder-based age informed network obtained from the cluster ECG results in better analysis of the health state of the subject. The three-step training strategy to initialize the K-means cluster centroids and optimizing the combined loss of reconstruction and cluster centroids resulted in better analysis of the biological age of the subject.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method, the method comprising:

receiving (302), via one or more hardware processors, a set of pre-processed ECG of a subject by a Deep Learning (DL) model wherein the DL model is an autoencoder comprising an encoder, and a decoder; and training (304), via one or more hardware processors, the DL model to generate a trained DL model by optimizing (a) one or more losses of an autoencoder enabled encoding-decoding, (b) one or more errors of age prediction, and (c) one or more errors of cluster prediction, by a three-step training, wherein

in a first step of the three-step training, a reconstruction loss associated with obtaining a first reconstructed ECG for the set of pre-processed ECG is optimized by iteratively updating randomly initialized one or more weights of the encoder, wherein for obtaining the first reconstructed ECG, the set of pre-processed ECG is encoded to a first set of latent code by the encoder, and the first set of latent code is decoded by the decoder, in a second step of the three step training, a reconstruction loss and an error of age prediction associated with obtaining a second reconstructed ECG are optimized by iteratively updating the one or more weights of the encoder, wherein obtaining the second reconstructed ECG comprises (a) re-initializing the one or more weights of the encoder from the first set of latent code to encode an age-informed second set of latent code; and (b) decoding the age-informed second set of latent code by the decoder, and wherein the age information is processed by a sigmoid layer, and

in a third step of the three step training, the reconstruction loss and an error of cluster prediction associated with obtaining (a) a third reconstructed ECG, and (b) one or more cluster centers, are optimized by iteratively updating the one or more weights of the encoder, wherein obtaining the third reconstructed ECG and the one or more cluster centers comprises re-initializing the one or more weights of the encoder from the age-informed second set of latent code for encoding the age-informed second set of latent code, and wherein the error of cluster prediction is associated with the extraction of the one or more clusters from the set of pre-processed ECG by a k-means clustering layer, and wherein the optimized reconstruction loss, the optimized error of age prediction, and the optimized error of cluster prediction causes reduction in a total loss in training the DL model.

2. The method as claimed in claim 1, wherein the pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time.

3. The method as claimed in claim 1, wherein the DL model is a two-dimensional autoencoder model having a configuration of the encoder and the decoder, wherein,

the encoder has a first Conv2D layer and a second Conv2D layer followed by a batch normalization (BN) and a dropout (DR), a third Conv2D followed by only the BN, a fourth Conv2D followed by the BN and the DR, a fifth Conv2D is Max-pooled (MP) after the BN, and a MP layer is flattened to generate a 1D latent code, and the decoder is a transposed Conv2D (Conv2D Trans), wherein a first layer in the decoder is a reshape (RE), a second layer is Upsample2D (US) followed by one or more layers that are opposite to the encoder part, a third layer is a dropout layer in addition to the encoder layer, and a final layer is Conv2D to generate reconstruction.

4. The method as claimed in claim 1, wherein the k-clustering layer replaces the sigmoid layer to extract the one or more clusters from the set of pre-processed ECG.

5. The method as claimed in claim 1, wherein the trained DL model provides one or more cluster assignments indicating relationship of a biological age (BA) of the subject with a measured ECG of the subject, wherein the BA of the subject is treated as a function of a chronological age (CA) and the ECG of the subject, and is represented as

$$=> (BA) = f_1 (ECG, CA)$$

$$=> BA = f_2 (f_1 (ECG) + (CA)),$$

where, $f_1$ (ECG) represent features extracted from the first reconstructed ECG and is equivalent to the first set of latent code, and
$f_2 (f_1 (ECG) + (CA))$ represent features extracted from the second reconstructed ECG and is equivalent to the age-informed second set of latent code.

6. The method as claimed in claim 1, wherein the total loss is a combination of a first combined loss and a second combined loss, wherein

the first combined loss is a summation of the reconstruction loss of the second reconstructed ECG and the error of age prediction, and
the second combined loss is a summation of the reconstruction loss of the third reconstructed ECG and the error of cluster prediction, wherein the total loss is represented as:

$$CL_1 = (1 - \lambda_1) MAE_{Recon} + \lambda_1 MAE_{Age}$$

$$CL_2 = (1 - \lambda_2)MAE_{Recon} + \lambda_2 MAE_{Clust}$$

and wherein,

$(1 - \lambda_1)MAE_{Recon}$ is a mean absolute error of reconstruction of the second reconstructed ECG,
$\lambda_1 MAE_{Age}$ is a mean prediction error of age,
$(1 - \lambda_2)MAE_{Recon}$ is a mean absolute error of reconstruction of the third reconstructed ECG, and
$\lambda_2 MAE_{Clust}$ is a mean absolute cluster centroids error ($MAE_{Clust}$).

7. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive a set of pre-processed ECG of a subj ect by a Deep Learning (DL) model wherein the DL model is an autoencoder comprising an encoder, and a decoder; and
train the DL model to generate a trained DL model by optimizing (a) one or more losses of an autoencoder enabled encoding-decoding, (b) one or more errors of age prediction, and (c) one or more errors of cluster prediction, by a three-step training, wherein

in a first step of the three-step training, a reconstruction loss associated with obtaining a first reconstructed ECG for the set of pre-processed ECG is optimized by iteratively updating randomly initialized one or more weights of the encoder, wherein for obtaining the first reconstructed ECG, the set of pre-processed ECG is encoded to a first set of latent code by the encoder, and the first set of latent code is decoded by the decoder,
in a second step of the three step training, a reconstruction loss and an error of age prediction associated with obtaining a second reconstructed ECG are optimized by iteratively updating the one or more weights of the encoder, wherein obtaining the second reconstructed ECG comprises (a) re-initializing the one or more weights of the encoder from the first set of latent code to encode an age-informed second set of latent code; and (b) decoding the age-informed second set of latent code by the decoder, and wherein the age information is processed by a sigmoid layer, and
in a third step of the three step training, the reconstruction loss and an error of cluster prediction associated with obtaining (a) a third reconstructed ECG, and (b) one or more cluster centers, are optimized by iteratively updating the one or more weights of the encoder, wherein obtaining the third reconstructed ECG and the one or more cluster centers comprises re-initializing the one or more weights of the encoder from the age-informed second set of latent code for encoding the age-informed second set of latent code, and wherein the error of cluster prediction is associated with the extraction of the one or more clusters from the set of pre-processed ECG by a k-means clustering layer, and wherein the optimized reconstruction loss, the optimized error of age prediction, and the optimized error of cluster prediction causes reduction in a total loss in training the **DL** model.

8. The system as claimed in claim 7, wherein the pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time.

9. The system as claimed in claim 7, wherein the DL model is a two-dimensional autoencoder model having a configuration of the encoder and the decoder, wherein,

the encoder has a first Conv2D layer and a second Conv2D layer followed by a batch normalization (BN) and a dropout (DR), a third Conv2D followed by only the BN, a fourth Conv2D followed by the BN and the DR, a fifth Conv2D is Max-pooled (MP) after the BN, and a MP layer is flattened to generate a 1D latent code, and
the decoder is a transposed Conv2D (Conv2D Trans), wherein a first layer in the decoder is a reshape (RE), a second layer is Upsample2D (US) followed by one or more layers that are opposite to the encoder part, a third layer is a dropout layer in addition to the encoder layer, and a final layer is Conv2D to generate reconstruction.

10. The system as claimed in claim 7, wherein the k-clustering layer replaces the sigmoid layer to extract the one or more clusters from the set of pre-processed ECG.

11. The system as claimed in claim 7, wherein the trained DL model provides one or more cluster assignments indicating relationship of a biological age (BA) of the subject with a measured ECG of the subject, wherein the BA of the subject is treated as a function of a chronological age (CA) and the ECG of the subject, and is represented as

$$=> (BA) = f_1 (ECG, CA)$$

$$=> BA = f_2 (f_1 (ECG) + (CA)),$$

where, $f_1$ (ECG) represent features extracted from the first reconstructed ECG and is equivalent to the first set of latent code, and
$f_2 (f_1 (ECG) + (CA))$ represent features extracted from the second reconstructed ECG and is equivalent to the age-informed second set of latent code.

12. The system as claimed in claim 7, wherein the total loss is a combination of a first combined loss and a second combined loss, wherein

the first combined loss is a summation of the reconstruction loss of the second reconstructed ECG and the error of age prediction, and
the second combined loss is a summation of the reconstruction loss of the third reconstructed ECG and the error of cluster prediction, wherein the total loss is represented as:

$$CL_1 = (1 - \lambda_1)MAE_{Recon} + \lambda_1 MAE_{Age}$$

$$CL_2 = (1 - \lambda_2)MAE_{Recon} + \lambda_2 MAE_{Clust}$$

and wherein,

$(1 - \lambda_1)MAE_{Recon}$ is a mean absolute error of reconstruction of the second reconstructed ECG,
$\lambda_1 MAE_{Age}$ is a mean prediction error of age,
$(1 - \lambda_2)MAE_{Recon}$ is a mean absolute error of reconstruction of the third reconstructed ECG, and
$\lambda_2 MAE_{Clust}$ is a mean absolute cluster centroids error ($MAE_{Clust}$).

13. One or more non-transitory machine-readable information storage mediums comprising one or more instructions which when executed by one or more hardware processors cause:

receiving, a set of pre-processed ECG of a subject by a Deep Learning (DL) model wherein the DL model is an autoencoder comprising an encoder, and a decoder; and
training, the DL model to generate a trained DL model by optimizing (a) one or more losses of an autoencoder enabled encoding-decoding, (b) one or more errors of age prediction, and (c) one or more errors of cluster prediction, by a three-step training, wherein

in a first step of the three-step training, a reconstruction loss associated with obtaining a first reconstructed ECG for the set of pre-processed ECG is optimized by iteratively updating randomly initialized one or more weights of the encoder, wherein for obtaining the first reconstructed ECG, the set of pre-processed ECG is encoded to a first set of latent code by the encoder, and the first set of latent code is decoded by the decoder, in a second step of the three step training, a reconstruction loss and an error of age prediction associated with obtaining a second reconstructed ECG are optimized by iteratively updating the one or more weights of the encoder, wherein obtaining the second reconstructed ECG comprises (a) re-initializing the one or more weights of the encoder from the first set of latent code to encode an age-informed second set of latent code; and (b) decoding the age-informed second set of latent code by the decoder, and wherein the age information is processed by a sigmoid layer, and
in a third step of the three step training, the reconstruction loss and an error of cluster prediction associated with obtaining (a) a third reconstructed ECG, and (b) one or more cluster centers, are optimized by iteratively updating the one or more weights of the encoder, wherein obtaining the third reconstructed ECG and the one or more cluster centers comprises re-initializing the one or more weights of the encoder from the age-informed second set of latent code for encoding the age-informed second set of latent code, and wherein the error of cluster prediction is associated with the extraction of the one or more clusters from the set of pre-processed ECG by a k-means clustering layer, and wherein the optimized reconstruction loss, the optimized error of age prediction, and the optimized error of cluster prediction causes reduction in a total loss in training the DL model.

**14.** The one or more non-transitory machine readable information storage mediums in claim 13, wherein the pre-processed ECG are obtained by normalizing one or more channels of the ECGs using a min-max normalization, wherein the one or more channels are individually normalized based on a minimum value and a maximum value and segmenting the pre-processed ECG into a plurality of overlapping windows for a specific period of time.

**15.** The one or more non-transitory machine readable information storage mediums in claim 13, wherein the DL model is a two-dimensional autoencoder model having a configuration of the encoder and the decoder, wherein,

the encoder has a first Conv2D layer and a second Conv2D layer followed by a batch normalization (BN) and a dropout (DR), a third Conv2D followed by only the BN, a fourth Conv2D followed by the BN and the DR, a fifth Conv2D is Max-pooled (MP) after the BN, and a MP layer is flattened to generate a 1D latent code, and
the decoder is a transposed Conv2D (Conv2D Trans), wherein a first layer in the decoder is a reshape (RE), a second layer is Upsample2D (US) followed by one or more layers that are opposite to the encoder part, a third layer is a dropout layer in addition to the encoder layer, and a final layer is Conv2D to generate reconstruction.

System **100**

Processors(s) **104**

I/O Interface(s) **106**

Memory **102**

Database **108**

Deep learning model 110

**FIG. 1**

FIG. 2

300

receiving (302), via one or more hardware processors, a set of pre-processed ECG of a subject by a Deep Learning (DL) model wherein the DL model is an autoencoder comprising an encoder, and a decoder ⌐ 302

generating (304), via the one or more hardware processors, a training data by optimizing (a) one or more losses of an autoencoder enabled encoding-decoding, (b) one or more errors of age prediction, and (c) one or more errors of cluster prediction, by a three-step training wherein,

in a first step, random initialization of encoder weights generate a first reconstructed ECG, and a reconstruction loss is optimized by iteratively updation of the encoder weights,

in a second step, re-initialization of the encoder weights from a first set of latent code generate a second reconstructed ECG via an age-informed second set of latent code, wherein the age information is processed by a sigmoid layer, and iterative updation of the encoder weights optimizes the reconstruction loss, and an error of age prediction, and

in a third step, re-initialization of the encoder weights from the age-informed second reconstructed ECG generate a third reconstructed ECG, wherein a k-clustering layer replaces the sigmoid layer prior to the re-initialization to form clusters, and iterative updation of the encoder weights optimizes the reconstruction loss, and an error of cluster prediction

⌐ 304

FIG. 3

FIG. 4

**FIG. 5A**

**FIG. 5B**

## EP 4 674 348 A1

Europäisches Patentamt
European Patent Office
Office européen des brevets

# EUROPEAN SEARCH REPORT

**Application Number**

EP 25 18 4339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2023 0155682 A (VUNO INC [KR]) 13 November 2023 (2023-11-13) * paragraphs [0021], [0022], [0044] - [0063], [0106] - [0117], [0130] * ----- | 1-15 | INV. A61B5/346 A61B5/00 |
| X | US 2022/249031 A1 (CLIFTON DAVID [GB] ET AL) 11 August 2022 (2022-08-11) * paragraphs [0001], [0005], [0069] - [0111] * ----- | 1-15 | |
| A | US 2021/361217 A1 (ATTIA ITZHAK ZACHI [US] ET AL) 25 November 2021 (2021-11-25) * paragraphs [0061] - [0066], [0078] * ----- | 1-15 | |
| A | PAIXÃO GABRIELA MIANA ET AL: "ECG-AGE FROM ARTIFICIAL INTELLIGENCE: A NEW PREDICTOR FOR MORTALITY? THE CODE (CLINICAL OUTCOMES IN DIGITAL ELECTROCARDIOGRAPHY) STUDY", JOURNAL OF THE AMERICAN COLLEGE OF CARDIOLOGY, ELSEVIER, AMSTERDAM, NL, vol. 75, no. 11, 16 March 2020 (2020-03-16), page 3672, XP086107003, ISSN: 0735-1097, DOI: 10.1016/S0735-1097(20)34299-6 [retrieved on 2020-03-16] * the whole document * ----- | 1-15 | TECHNICAL FIELDS SEARCHED (IPC) A61B |
| A | KR 2022 0067724 A (ULSAN NAT INST SCIENCE & TECH UNIST [KR]; BIO AGE CO LTD [KR]) 25 May 2022 (2022-05-25) * paragraphs [0007], [0017], [0018], [0029] - [0034], [0068] * ----- | 1-15 | |

The present search report has been drawn up for all claims

| Place of search | Date of completion of the search | Examiner |
|---|---|---|
| Berlin | 20 November 2025 | Pohjamo, Terhi |

EPO FORM 1503 03.82 (P4C01)

## ANNEX TO THE EUROPEAN SEARCH REPORT
## ON EUROPEAN PATENT APPLICATION NO.

EP 25 18 4339

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2025

| Patent document cited in search report | | Publication date | Patent family member(s) | | Publication date |
|---|---|---|---|---|---|
| KR 20230155682 A | | 13-11-2023 | EP | 4483808 A1 | 01-01-2025 |
| | | | JP | 2025514694 A | 09-05-2025 |
| | | | KR | 20230155682 A | 13-11-2023 |
| | | | US | 2025191758 A1 | 12-06-2025 |
| | | | WO | 2023214647 A1 | 09-11-2023 |
| US 2022249031 A1 | | 11-08-2022 | EP | 4003147 A1 | 01-06-2022 |
| | | | US | 2022249031 A1 | 11-08-2022 |
| | | | WO | 2021014150 A1 | 28-01-2021 |
| US 2021361217 A1 | | 25-11-2021 | US | 2021361217 A1 | 25-11-2021 |
| | | | WO | 2019140217 A1 | 18-07-2019 |
| KR 20220067724 A | | 25-05-2022 | NONE | | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421051386